# EUROPÄISCHE PATENTSCHRIFT

(11) **EP 1 006 915 B1**
(45) Veröffentlichungstag und Bekanntmachung des Hinweises auf die Patenterteilung: **21.05.2003**
(21) Anmeldenummer: 97936555.8
(22) Anmeldetag: 04.09.1997
(51) Int. Cl.: A61B 17/80

(54) **SYMMETRISCHE KNOCHENPLATTE**
SYMMETRICAL BONE PLATE
PLAQUE SYMETRIQUE POUR OSTEOSYNTHESE

(43) Veröffentlichungstag der Anmeldung: 14.06.2000
(73) Patentinhaber: SYNTHES AG Chur, 7002 Chur (CH)
(72) Erfinder: KLAUE, Kaj, CH-3005 Bern (CH)
(74) Vertreter: Lusuardi, Werther Giovanni, Dr.
(86) Internationale Anmeldenummer: CH9700323
(87) Internationale Veröffentlichungsnummer: WO99011188

(56) Entgegenhaltungen:
- WO-A-90/07304
- FR-A- 2 642 958
- FR-A- 2 680 673
- US-A- 3 463 148

## Beschreibung

Die Erfindung betrifft eine Knochenplatte gemäss dem Oberbegriff des Patentanspruchs 1.
Aus der FR-A-2 642 958 ist formal eine gattungsgemässe Knochenplatte bekannt, wobei allerdings die Vorteile der symmetrischen Ausgestaltung nicht erkannt wurden; die darin als bevorzugte Ausführungsform beschriebene Platte weist eine Längskrümmung auf und ist somit unsymmetrisch ausgebildet.
Aus der FR-A-2 680 673 ist formal ebenfalls eine gattungsgemässe Knochenplatte bekannt. Die in den Zeichnungen symmetrisch dargestellte Knochenplatte widerspricht der in der Beschreibung angegeben Verwendungsart für die Lendenwirbelsäule, welche eine anatomische Krümmung von ungefähr 158° verlangt. Auch hier wurden somit die Vorteile der symmetrischen Ausgestaltung nicht erkannt.

Die Nachteile der unsymmetrischen Ausgestaltung von Knochenplatten besteht darin, dass solche Platten unbedingt mit der richtigen, d.h. mit ihrer Unterseite auf den Knochen geschraubt werden müssen, um optimale Resultate zu erzielen. Die klinische Erfahrung zeigt nun aber, dass es besonders bei Miniplatten und in der Eile zu Verwechslungen kommt, so dass Knochenplatten mit der falschen, d.h. mit ihrer Oberseite auf den Knochen appliziert werden.

Ein weiterer Nachteil der aus den FR-A-2 642 958 und FR-A-2 680 673 bekannten Knochenplatten liegt in ihrem rechteckigen Profil

Hier will die Erfindung Abhilfe schaffen. Der Erfindung liegt das Problem zugrunde eine Knochenplatte zu schaffen, welche mit identischem klinischem Resultat sowohl mit ihrer Unterseite, als auch mit ihrer Oberseite auf den Knochen schraubbar ist. Für spezielle klinische Situationen soll auch die Möglichkeit bestehen dieselben Platte am einen Ende mit der Oberseite und am anderen Ende mit der Unterseite an zwei Knochenfragmenten zu befestigen. Zusammen mit den Schrauben erscheint dann die Platte als "Z". Eine solche Anwendung ist dann von Interesse, wenn man die Hälfte der Platte in einem Segment entweder vollständig einstaucht und das andere Segment auf der Aussenseite der Corticalis befestigt oder bei einer Osteotomie eine Verschiebung um eine gesamt Knochenbreite erzielen will. Damit dient das Implantat als extra-medulläre und intra-medulläre Vorrichtung (Nagel + Platte).

Die Erfindung löst die gestellte Aufgabe mit einer Knochenplatte, welche die Merkmale des Anspruchs 1 aufweist.

Dank der symmetrischen Ausbildung der erfindungsgemässen Knochenplatte können keine Verwechslungen der Ober- mit der Unterseite mehr erfolgen, was die Sicherheit in der Anwendung erheblich erhöht.

Eine bevorzugte Weiterbildung der Erfindung besteht darin, dass die Plattenbohrungen kreiszylindrisch ausgebildet sind; sie hat den Vorteil, dass der geschwächte Teil der Platte minimalisiert ist.

Bei einer anderen Ausführungsform erweitern sich die Plattenbohrungen gegen die beiden Oberflächen hin, vorzugsweise in Form eines Konus. Sie hat den Vorteil, dass der Schraubenkopf versenkt werden kann und die Lochkante minimalisiert wird.

Das orthogonal zur Längsachse verlaufende Profil der erfindungsgemässen Knochenplatte ist oval.

Im Bereich zwischen den Plattenbohrungen können in Richtung der Längsachse gesehen die beiden Oberflächen auch konkav ausgebildet sind. Bei einer konkaven Ausbildung kann der zusätzliche Vorteil erreicht werden, dass die Steifigkeit des Implantats im Bereich der Plattenlöcher und im Zwischenlochbereich gleich gross ist. Damit wird erreicht, dass die Steifigkeit proportional zur Festigkeit gegen Ermüdung ist.

Geringfügige Abweichungen von der Symmetrie der durch die Symmetrieebene begrenzten Hälften A und B der Knochenplatte sind zwar zulässig. doch sollten die geringfügig unterschiedlichen Volumina Vₐ und V_{b} im Bereich von 0,98 < Vₐ/V_{b} < 1,02, vorzugsweise von 0,99 < Vₐ/V_{b} < 1,01 liegen.

Die durch die Erfindung erreichten Vorteile sind im wesentlichen darin zu sehen, dass dank der erfindungsgemässen Knochenplatte:
- eine hohe Versatilität erzielt wird;
- mit dem gleichen Implantat ein Maximum an verschiedenen Knochenbrüchen behandelt werden können;
- die Konstruktion des Implantats vereinfacht ist; und
- die Schrauben zur Fixation und die Schrauben zur Verriegelung der Platte gleich sind.

Die Erfindung und Weiterbildungen der Erfindung werden im folgenden anhand der teilweise schematischen Darstellungen mehrerer Ausführungsbeispiele noch näher erläutert.
Es zeigen:
Fig. 1 eine perspektivische Ansicht einer Knochenplatte mit rechteckigem Profil, welches nicht die Erfindung darstellt.
Fig. 2 einen Längsschnitt durch eine erfindungsgemässe Knochenplatte;
Fig. 3 einen Längsschnitt durch eine erfindungsgemässe Knochenplatte mit leicht angesenkten Plattenbohrungen;
Fig. 4 einen Längsschnitt durch eine erfindungsgemässe Knochenplatte mit stark angesenkten Plattenbohrungen und zwei Typen von Schrauben;
Fig. 5 perspektivische Ansicht einer erfindungsgemässen Knochenplatte mit ovalem Profil.

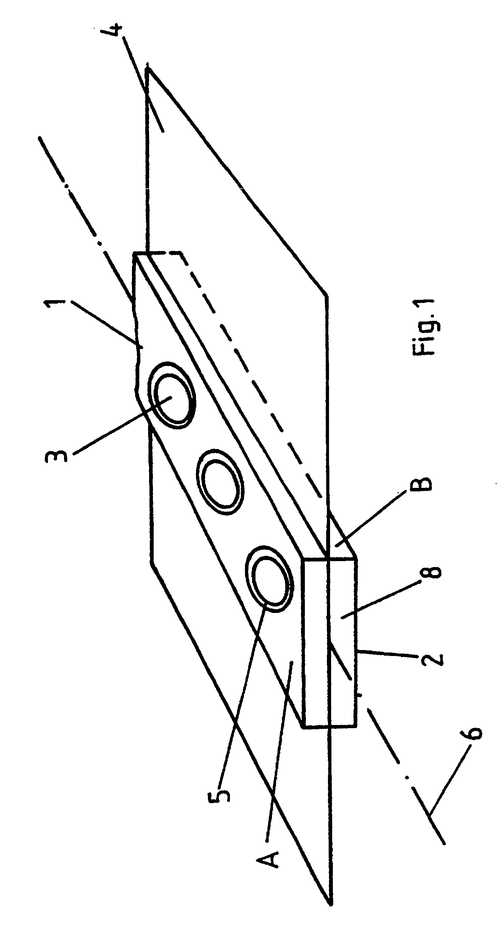

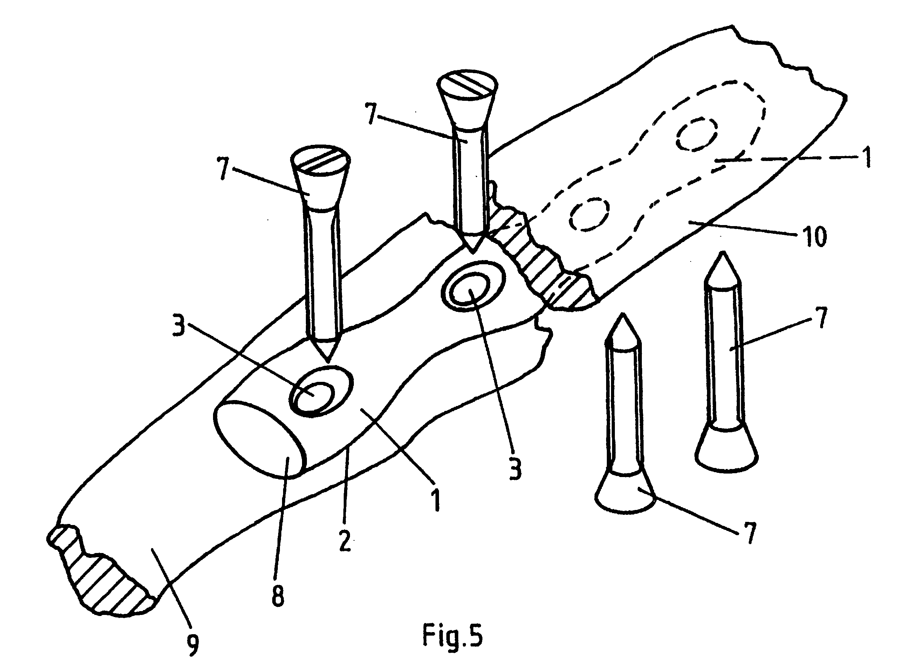

Die in Fig. 1 dargestellte Knochenplatte weist eine Längsachse 6, zwei Oberflächen 1,2 sowie mehrere, die beiden Oberflächen 1,2 verbindende Plattenbohrungen 3 auf. Die Plattenbohrungen 3 dienen der Aufnahme von Knochenschrauben 7 (Fig. 5). Zwischen den beiden Oberflächen 1 und 2 verläuft die Mittelebene 4, welche die Knochenplatte in zwei im wesentlichen symmetrische Hälften A und B aufteilt.

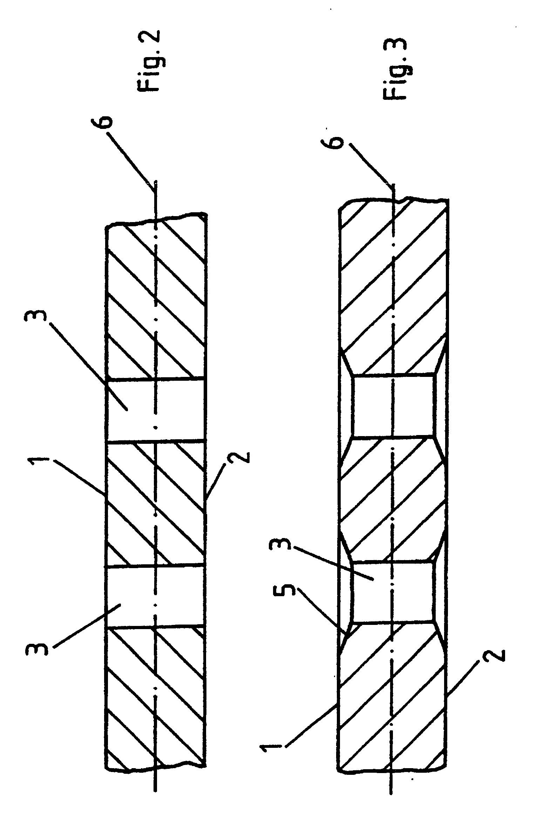

Wie in Fig. 2 dargestellt können die Plattenbohrungen 3 kreiszylindrisch ausgebildet sein.

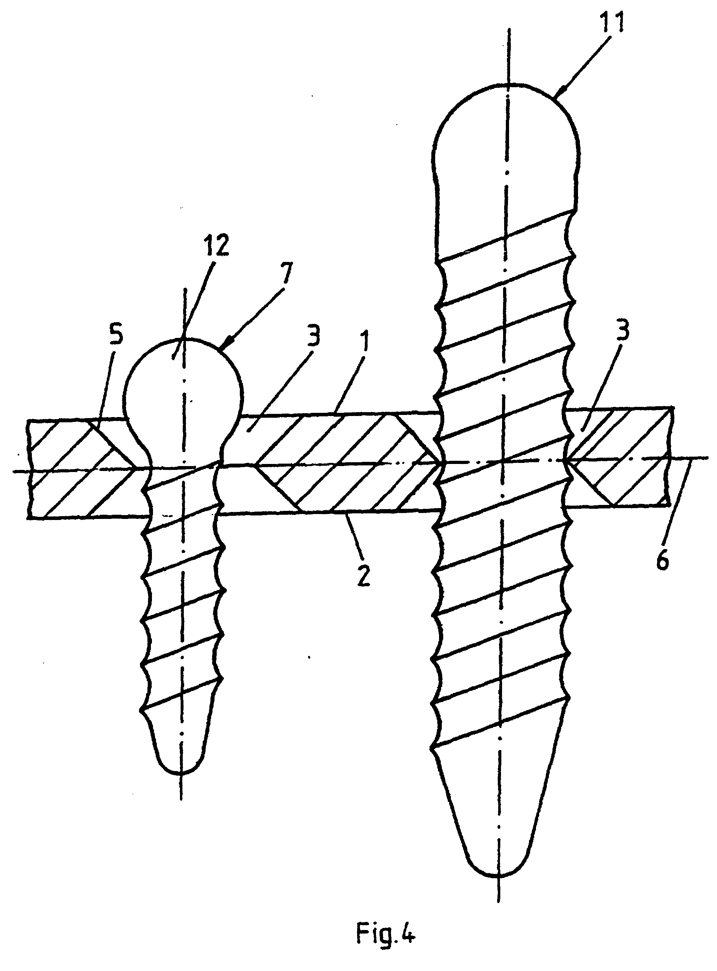

Bei anderen Ausführungsformen, z.B. gemäss den Fig. 3 und 4, sind die Plattenbohrungen 3 gegen die beiden Oberflächen 1,2 hin erweitert, und zwar in Form eines Konus 5. Das orthogonal zur Längsachse 6 verlaufende Profil 8 ist - wie in Fig. 1 gezeigt - rechteckig. Bei der Erfindung ist es, wie in Fig. 5 dargestellt, oval.

Bei der in Fig. 5 dargestellten Ausführungsform sind die beiden Oberflächen 1 und 2 - im Bereich zwischen den Plattenbohrungen 3 - konkav ausgebildet, so dass eine gleichförmige Steifigkeit der Knochenplatte erreicht wird.

In Fig. 4 sind zwei Typen von Knochenschrauben 7 und 11 mit unterschiedlichen Funktionen dargestellt. Bei der links gezeigten Knochenschraube 7 handelt es sich um eine Schraube zur Fixation der Knochenplatte; der Durchmesser ihres Gewindes ist kleiner als die engste Stelle der Plattenbohrung 3, so dass es nicht mit der Plattenbohrung 3 in Eingriff kommt. Bei der rechts gezeigten Knochenschraube 11 handelt es sich um eine Schraube zur Verriegelung der Knochenplatte; der Durchmesser ihres Gewindes ist grösser als die engste Stelle der Plattenbohrung 3 so dass es seitlich in die Plattenbohrung eingreift.
Der Kopf 12 der Knochenschraube 7 ist kugelig ausgebildet, damit er auf dem als Gleitbahn dienenden Konus 5 der Plattenbohrung 3 optimal gleiten kann.

In Fig. 5 ist eine Anwendung der Knochenplatte gezeigt, bei welcher der vordere Teil mit der einen Oberfläche 2 am vorderen Knochenfragment 9 mittels der beiden Knochenschrauben 7 befestigt wird, und der hintere Teil mit der anderen Oberfläche 1 am hinteren Knochenfragment 10 mit zwei Knochenschrauben 7 befestigt wird.

## Patentansprüche

1. Knochenplatte mit einer Längsachse (6), zwei Oberflächen (1,2) sowie mehreren, die beiden Oberflächen (1,2) 'verbindenden Plattenbohrungen (3) zur Aufnahme von Knochenschrauben, wobei die Knochenplatte bezüglich einer zwischen den beiden Oberflächen (1,2) verlaufenden Mittelebene (4) im wesentlichen symmetrisch ist,
**dadurch gekennzeichnet, dass**
das orthogonal zur Längsachse (6) verlaufende Profil (8) oval ist.

2. Knochenplatte nach Anspruch 1, **dadurch gekennzeichnet, dass** die Plattenbohrungen (3) kreiszylindrisch ausgebildet sind.

3. Knochenplatte nach Anspruch 1 oder 2, **dadurch gekennzeichnet, dass** sich die Plattenbohrungen (3) gegen die beiden Oberflächen (1,2) hin erweitern, vorzugsweise in Form eines Konus (5).

4. Knochenplatte nach einem der Ansprüche 1 bis 3, **dadurch gekennzeichnet, dass** der Durchmesser mindestens einer der Plattenbohrungen (3) in Richtung der Längsachse (6) gemessen grösser ist als senkrecht zur Längsachse (6), d.h. eine ovale Form aufweist.

5. Knochenplatte nach einem der Ansprüche 1 bis 4, **dadurch gekennzeichnet, dass** im Bereich zwischen den Plattenbohrungen (3) die beiden Oberflächen (1,2) entlang der Längsachse (6) konkav ausgebildet sind.

6. Knochenplatte nach einem der Ansprüche 1 bis 5, **dadurch gekennzeichnet, dass** die durch die Symmetrieebene (4) begrenzten Hälften A und B der Knochenplatte geringfügig unterschiedliche Volumina Vₐ und V_{b} im Bereich von 0,98 < Vₐ/V_{b} < 1,02, vorzugsweise von 0,99 < Vₐ/V_{b} < 1,01 aufweisen.

## Claims

1. Bone plate having a longitudinal axis (6), two surfaces (1, 2) and a plurality of plate bores (3) connecting the two surfaces (1, 2) for accommodating bone screws, wherein the bone plate is essentially symmetrical with a middle plane (4) running between the two surfaces (1, 2),
**characterized in that**
the profile (8) running perpendicular to the longitudinal axis (6) is oval.

2. Bone plate according to claim 1, **characterized in that** the plate bores (3) are circular cylindrical in design.

3. Bone plate according to claim 1 or 2, **characterized in that** the plate bores (3) become wider toward the two surfaces (1, 2), preferably in the form of a cone (5).

4. Bone plate according to one of claims 1 through 3, **characterized in that** the diameter of at least one of the plate bores (3) as measured in the direction of the longitudinal axis (6) is greater than that perpendicular to the longitudinal axis (6), i.e. has an oval shape.

5. Bone plate according to one of claims 1 through 4, **characterized in that** the two surfaces (1, 2) are designed to be concave along the longitudinal axis (6) in the area between the plate bores (3).

6. Bone plate according to one of claims 1 through 5, **characterized in that** halves A and B of the bone plate bordered by the plane of symmetry (4) have slightly different volumes Vₐ and V_{b} in the range of 0.98 < Vₐ/V_{b} < 1.02, preferably in the range of 0.99 < Vₐ/V_{b} < 1.01.

## Revendications

1. Plaque pour ostéosynthèse présentant un axe longitudinal (6), deux surfaces (1, 2) ainsi que plusieurs alésages de plaque (3) qui relient les deux surfaces (1, 2), pour la réception de vis pour ostéosynthèse, la plaque pour ostéosynthèse étant essentiellement symétrique par rapport à un plan central (4) qui s'étend entre les deux surfaces (1, 2),
**caractérisée en ce que** le profil (8) qui s'étend perpendiculairement à l'axe longitudinal (6) est ovale.

2. Plaque pour ostéosynthèse selon la revendication 1, **caractérisée en ce que** les alésages de plaque (3) présentent une configuration cylindrique circulaire.

3. Plaque pour ostéosynthése selon la revendication 1 ou 2, **caractérisée en ce que** les alésages de plaque (3) s'évasent en direction des deux surfaces (1, 2), de préférence sous la forme d'un coude (5).

4. Plaque pour ostéosynthèse selon l'une des revendications 1 à 3, **caractérisée en ce que** les diamètres d'au moins l'un des alésages de plaque (3) est plus grand dans la direction de l'axe longitudinal (6) que perpendiculairement à l'axe longitudinal (6), c'est-à-dire présente une forme ovale.

5. Plaque pour ostéosynthèse selon l'une des revendications 1 à 4, **caractérisée en ce que** dans la zone située entre les alésages de plaque (3), les deux surfaces (1, 2) présentent une configuration concave le long de l'axe longitudinal (6).

6. Plaque pour ostéosynthèse selon l'une des revendications 1 à 5, **caractérisée en ce que** les moitiés A et B de la plaque pour ostéosynthèse sont limitées à 10,98 < Vₐ et V_{b} < 1,02 et de préférence à 0,99 < Vₐ et V_{b} < 1,01.
